# EUROPÄISCHE PATENTSCHRIFT

(11) **EP 1 031 054 B1**
(45) Veröffentlichungstag und Bekanntmachung des Hinweises auf die Patenterteilung: **21.05.2003**
(21) Anmeldenummer: 98956908.2
(22) Anmeldetag: 12.11.1998
(51) Int. Cl.: G02B 7/02, G02B 23/24

(54) **VERFAHREN ZUM MONTIEREN VON STABLINSEN IN EINEM ENDOSKOP SOWIE ENDOSKOP MIT SOLCHEN STABLINSEN**
METHOD FOR MOUNTING ROD LENSES IN AN ENDOSCOPE AND ENDOSCOPE WITH SAID ROD LENSES
PROCEDE DE MONTAGE DE LENTILLES EN FORME DE BARRES DANS UN ENDOSCOPE ET ENDOSCOPE DOTE DE TELLES LENTILLES EN FORME DE BARRES

(30) Priorität: 15.11.1997 DE 19750685
(43) Veröffentlichungstag der Anmeldung: 30.08.2000
(73) Patentinhaber: Karl Storz GmbH & Co. KG, 78532 Tuttlingen (DE)
(72) Erfinder: RUDISCHHAUSER, Jürgen, D-78532 Tuttlingen (DE); KEHR, Ulrich, D-73760 Ostfildern (DE)
(74) Vertreter: Weller, Wolfgang, Dr.
(86) Internationale Anmeldenummer: EP9807252
(87) Internationale Veröffentlichungsnummer: WO99026096

(56) Entgegenhaltungen:
- DE-A- 3 431 631
- DE-U- 9 417 262
- US-A- 4 148 550
- US-A- 4 148 551

## Beschreibung

Die vorliegende Erfindung betrifft ein Verfahren zum Montieren zumindest einer Stablinse im Inneren eines zu einem Endoskop gehörenden Rohrs durch Fixieren der Stablinse im Inneren des Rohrs an zumindest zwei axial voneinander beabstandeten Stellen, mit folgenden Schritten:
- Auswählen des Außendurchmessers der Stablinse derart, daß nach Einbringen der Stablinse das Rohr zwischen der Außenseite der Stablinse und der Innenseite des Rohrs umfänglich ein Luftspalt entsteht, und
- Positionieren der Außenseite der Stablinse in einem definierten radialen Abstand zu der Innenseite des Rohrs durch zumindest zwei Zentrierhilfen, die an zumindest zwei axial voneinander beabstandeten Stellen der Außenseite der Stablinse angreifen, wobei jede Zentrierhilfe Zentrierelemente aufweist, welche gleichmäßig um den Umfang der Stablinse verteilt sind.

Die Erfindung betrifft ferner ein Endoskop mit einem Rohr, in dessen Innerem zumindest eine Stablinse an zumindest zwei axial voneinander beabstandeten Stellen fixiert ist, wobei die Stablinse einen Außendurchmesser aufweist, der derart bemessen ist, daß nach Einbringen der Stablinse in das Rohr zwischen der Außenseite der Stablinse und der Innenseite des Rohrs ein Luftspalt vorhanden ist, und wobei die Außenseite der Stablinse in einem definierten radialen Abstand zu der Innenseite des Rohrs angeordnet ist.

Ein derartiges Verfahren und ein entsprechendes Endoskop sind aus der DE 34 31 631 C2 bekannt.

Endoskope werden allgemein in der Chirurgie dazu verwendet, Körperhohlräume und Hohlorgane zu inspizieren. Endoskope weisen einen langerstreckten Rohrschaft auf, in dem Bauelemente eines optischen Beobachtungssystems angeordnet sind. Ferner sind im Inneren des Schafts weitere Vorrichtungen, meist eine Lichtzuführvorrichtung, und ggf. Kanäle für Instrumente, Spülflüssigkeit oder dergleichen vorgesehen. Die Lichtzuführung erfolgt meist über lichtleitende Glasfasern.

Das optische System starrer Endoskope ist meist in einem innerhalb des Endoskopschafts liegenden Rohr aufgenommen. Wesentliche Bestandteile des optischen Systems sind die Linsen, wobei insbesondere Stablinsen der sogenannten HOPKINS-Optik bei Endoskopen eingesetzt werden. Die Linsen sowie weitere Bestandteile des optischen Systems wie Blenden, Filter und dergleichen sind entlang der Rohrschaftachse angeordnet und haben die Aufgabe, eine klare Abbildung eines möglichst großen Blickfelds mit großer Auflösung und hohem Kontrast wiederzugeben.

Die wichtigste Voraussetzung hierfür ist die präzise Anordnung der Linsen und der weiteren Bauelemente des optischen Systems untereinander im Inneren des zu dem Endoskop gehörenden Rohrs. Die relative Position einer Linse ist nicht variabel, sondern genau definiert. Jede Verschiebung der Linsen relativ zueinander führt zu einer verminderten Bildqualität. Eine präzise Anordnung wird häufig dadurch erreicht, daß der Außendurchmesser einer Stablinse genau dem lichten Innendurchmesser des Rohres entspricht, die Linse also ohne radiales Spiel aufgenommen ist.

Zwischen den Linsen eingelegte Abstandshülsen samt einem distalen und einem proximalen Abschlußstück sorgen für eine axiale Positionierung und Fixierung der Linsen bzw. optischen Bauelemente.

Ein Problem liegt nun darin, daß bei der Handhabung des Endoskops sowie insbesondere bei seiner Reinigung Erschütterungen auftreten, bspw. bei einer Ultraschallreinigung, die dazu führen, daß die Linsen sich radial innerhalb des vorhandenen Spiels verschieben und insbesondere an den Kontaktflächen zwischen der Außenseite der Stablinsen und der Innenseite des Innenrohrs Abrieb entsteht, der die optische Abbildungsqualität beeinträchtigt.

Endoskopschäfte sind gegenüber ihrem Durchmesser sehr lang, übliche Durchmesser liegen im Bereich von einigen Millimetern, die Länge im Bereich von mehreren Dezimetern. Bei der Handhabung von starren Endoskopen wirken Biegemomente auf die dünnen und langen Schäfte ein, die dazu führen können, daß die relativ langen Stablinsen aus Glasmaterialien brechen.

Eine Lösung, wie Stablinsen im Inneren eines Endoskops unter Erhalt einer gewissen Flexibilität fixiert werden können, ist in der US 4,148,550 beschrieben, die der DE-OS 28 12 369 entspricht.

Die dort vorgesehenen Stablinsen sind im Inneren des Endoskopschafts durch ein zusätzliches inneres Rohr miteinander verbunden. Dieses innere Rohr weist in dem Bereich, in dem es die Enden zweier benachbarter, voneinander beabstandeter Stablinsen überbrückt, sich längserstreckende Schlitze auf.

Die Stablinsen sitzen dabei ohne radiales Spiel passend im inneren Rohr, d.h. der Außendurchmesser einer Linse entspricht dem lichten Innendurchmesser des inneren Rohrs. Die Stablinsen werden in dem Rohr entweder nur festgeklemmt oder durch Klebstoff verklebt. Dazu wird vorgeschlagen, bspw. einen durch Wärmebehandlung oder UV-Bestrahlung aktivierbaren Klebstoff zu verwenden.

Die Möglichkeit, den Rohrschaft in gewissen Grenzen zu verbiegen, ohne dadurch die Stablinsen zu brechen, wird durch die Schlitze des inneren Rohrs erreicht. Das innere Rohr kann im Bereich der Schlitze etwas abknicken, also im Bereich zwischen zwei benachbarten Stablinsen.

Dadurch, daß eine Biegsamkeit nur im Bereich der Schlitze des inneren Rohrs möglich ist, wird der minimal mögliche Biegeradius sehr groß. Dies gilt insbesondere dann, wenn die Abstände zwischen den Stablinsen im Verhältnis zu der Stablinsenlänge sehr klein sind. Ein gleichmäßiges bogenhaftes Krümmen des Rohrschafts ist nicht möglich. Wirken Biegemomente direkt im Bereich der Stablinse ein, die zu einem Verbiegen des äußeren Endoskopschafts führen, kann die Stablinse dennoch brechen. Solche punktuell einwirkenden Biegemomente können auftreten, wenn ein Endoskopschaft beim Handhaben auf eine Kante stößt oder herabfällt.

In der DE 44 38 511 A1 ist ein Endoskop beschrieben, bei dem eine Beschädigung der Stablinsen dadurch verhindert werden soll, daß diese innerhalb des Rohrs koaxial von einem Verstärkungsglied umgeben sind, das im Gegensatz zu der Stablinse aus einem Material mit hoher Festigkeit wie Metall oder Keramik herstellt ist. Dabei ist das Verstärkungsglied kürzer als die Stablinse selbst. Hierbei ergibt sich wiederum die Schwierigkeit, daß mit zunehmender Länge des Verstärkungsgliedes ein gleichmäßiges bogenhaftes Krümmen des Rohrschafts verhindert wird. Je kürzer das Verstärkungsglied jedoch gewählt wird, desto instabiler in bezug auf ein Verkippen ist die Stablinse innerhalb des Rohrs gelagert. Ziel des Verstärkungsgliedes ist es auch nicht, der radialen Abstand zwischen der Stablinse und dem Rohr zu vergrößern. Vielmehr soll durch das Verstärkungsglied der zentrale Bereich der Stablinse, in dem sich die Spannung bei einer Biegebeanspruchung konzentriert, geschützt werden.

In der bereits eingangs genannten DE 34 31 631 C2 ist eine Stablinsenhalterung beschrieben, bei der die Stablinsen mittels zweier, axial voneinander beabstandeter Distanzringe innerhalb des Rohrs gelagert sind. Hierdurch befinden sich die Stablinsen in einem radialen Abstand zur Innenfläche des Rohrs. Aufgrund dieses Abstands besteht ein Spielraum, so daß sich das Rohr bei einer Biegebeanspruchung unabhängig von der Stablinse verbiegen kann. Gemäß dieser Schrift sind die Distanzringe jedoch aus Materialien wie z.B. Metall, insbesondere Messing oder Kunststoff, insbesondere Hartkunststoff und dergleichen hergestellt. Aufgrund einer Verwendung derartig harter Materialien sind die Stablinsen trotz des bestehenden radialen Abstands sehr starr mit dem Rohr verbunden. Dementsprechend werden auch Biegebeanspruchungen des Rohrs weitgehend ungedämpft an die Stablinsen übertragen. Somit ist eine Beschädigung der Stablinsen durch Biegemomente trotz des bestehenden radialen Abstands nicht zuverlässig verhindert.

Aus einer Veröffentlichung in den OE-Reports No. 67, Juli 1989, Seite 13, published by SPIE, ist es bekannt, Linsen in einer Fassung unter Verwendung von nach dem Kleben zu entfernenden Zentrierelementen in Form von Zentrierschrauben einzukleben.

Aus der DE 37 30 094 C2 sind Zentrierelemente bekannt, welche gleichmäßig um den Umfang einer Linse verteilt sind.

Aus der DE-A-722 587 sind zwei axial voneinander beabstandete Zentrierhilfen bekannt und aus der DD 264 523 A1 sind Methoden zum koaxialen Einkleben von Linsen bekannt. Aus der DE 94 17 262 U und der US 4 148 551 A ist es bekannt, eine Stablinse in einem rohrförmigen Verstärkungsglied einzukleben.

Vor dem Hintergrund dieses Standes der Technik ist es Aufgabe der vorliegenden Erfindung, ein Verfahren sowie ein Endoskop der eingangs genannten Art zu schaffen, bei dem eine Übertragung von Biegekräften auf die zumindest eine Stablinse noch weiter verringert ist, während die Stablinse gleichzeitig sicher und lagegerecht im Inneren des Rohrs fixiert ist.

Diese Aufgabe wird bei dem eingangs genannten Verfahren durch ein Bewerkstelligen von direkten elastischen Klebeverbindungen zwischen der Außenseite der Stablinse und der Innenseite des Rohrs an den axial voneinander beabstandeten Stellen gelöst.

Die Aufgabe wird ferner bei dem eingangs genannten Endoskop dadurch gelöst, daß die Außenseite der Stablinse durch direkte elastische Klebestellen mittels Klebstoff an der Innenseite des Rohrs an den voneinander beabstandeten Stellen fixiert ist, wobei der Raum zwischen diesen Stellen frei von Klebstoff ist.

Bei dem erfindungsgemäßen Verfahren ist zunächst einmal vorteilhaft, daß das Einführen der Stablinse in das Rohr aufgrund des geringeren Außendurchmessers der Stablinse im Vergleich zu dem Innendurchmesser des Rohrs mühelos möglich ist. Das vorgesehene Rohr kann dabei der Rohrschaft des Endoskops selbst oder aber ein weiteres Innenrohr des Endoskops sein, das erst nach der Montage der Linsen in den Rohrschaft des Endoskops eingeführt wird.

Das Positionieren der Stablinse zentrisch in dem Rohr mit Hilfe der Zentrierhilfen stellt sicher, daß zwischen der Außenseite der Stablinse und der Innenseite des Rohrs ein gleichmäßiger Luftspalt, d.h. ein definierter radialer Abstand entsteht. Durch die axiale Beabstandung der Zentrierstellen, an denen die Zentrierhilfen angreifen, wird ein Verkippen der Stablinse innerhalb des Rohrs während der Montage sicher verhindert, so daß die Stablinse exakt längs der optischen Achse ausgerichtet werden kann. Je nach der Länge der zu montierenden Stablinse können hier zwei, drei oder auch mehr Zentrierhilfen vorgesehen werden, die jeweils axial voneinander beabstandet sind.

Nach dem Zentrieren der Stablinse wird eine elastische Klebeverbindung zwischen der Außenseite der Stablinse und der Innenseite des Rohrs bewerkstelligt, wobei der hier zu verwendende Klebstoff den Luftspalt nicht vollständig ausfüllt, sondern lediglich an zumindest zwei axial voneinander beabstandeten Klebestellen aufgebracht wird. Auch hier können je nach Stablinsenlänge mehrere jeweils in axialen Abständen zueinander liegende Klebestellen vorgesehen werden.

Der durch das erfindungsgemäße Verfahren erzeugte Zusammenbau aus einer oder mehreren Stablinsen im Inneren des Rohrs hat den Vorteil, daß die Stablinsen durch die Klebestellen sowohl gegen axiale als auch gegen radiale Verschiebung, insbesondere gegen Verkippung, gesichert sind. Durch das Anbringen des Klebstoffs an den Klebestellen wird somit für eine lagegerechte Fixierung der Stablinsen gesorgt. Sie liefern daher eine gleichbleibende Abbildungsqualität.

Durch den vorgesehenen Luftspalt hat die Außenseite der Stablinse mit der Innenseite des Rohrs keinen direkten Kontakt, so daß auch bei hoher Schüttelbeanspruchung (wie bspw. bei der Reinigung des Endoskops durch Ultraschall) kein Abrieb erzeugt wird, der die Optik negativ beeinträchtigen könnte.

Der Luftspalt sorgt darüber hinaus zusammen mit den erfindungsgemäßen Klebestellen für die gewünschte Flexibilität des Zusammenbaus aus Rohr und Linse gegenüber Belastungen, insbesondere gegenüber Biegemomenten. Bei einem Abbiegen des Rohrs erlaubt der Luftspalt es nämlich zusammen mit den Klebestellen, die eine elastische Verbindung zwischen Linse und Rohr herstellen, daß zunächst nur das Rohr alleine sich biegt. So wird auf die aus Glas bestehende und damit nur begrenzt belastbare Stablinse zunächst nur ein geringes Biegemoment ausgeübt, das sich erst dann erhöht, wenn das Abbiegen des Rohrs so groß wird, daß seine Innenseite die Außenseite der Stablinse berührt.

Das Rohr kann sich somit bogenförmig abbiegen, da hierfür ein Spielraum zwischen Stablinse und Rohr in Form des Luftspalts vorhanden ist.

So wird ein Zerbrechen oder Zersplittern der Stablinse unter den üblichen Belastungen eines starren Endoskops, die sowohl während der Handhabung, z.B. während der Operation oder beim Ablegen des Endoskops, als auch während der Reinigung, bspw. durch Kontakt mit weiteren zu reinigenden Geräten, auf den Endoskopschaft ausgeübt werden, sicher verhindert.

Der Luftspalt sorgt zusammen mit den Klebestellen zusätzlich für einen Schutz der Linse gegenüber äußeren Krafteinwirkungen, da die Klebeverbindung elastisch ist und Stöße daher dämpfen kann. So wird die Gefahr eines Zerbrechens der Stablinsen auch bei einem heftigen Ablegen oder sogar Herabfallen des Endoskops erheblich verringert.

Das erfindungsgemäße Endoskop hat den Vorteil, daß seine Stablinsen sicher fixiert sind, so daß axiale oder radiale Verschiebungen verhindert werden. Gleichzeitig ist eine Biegung des Rohrschafts des Endoskops in gewissem Ausmaß möglich, und zwar dadurch, daß zwischen der Außenseite der Stablinse und der Innenseite des Rohrs ein Luftspalt vorgesehen ist, der nur von den elastischen Klebestellen überbrückt wird. So kann das Rohr sich in einem gewissen Umfang verbiegen, ohne daß die Biegemomente auf die Stablinse einwirken. Dadurch wird ein Zerbrechen der Stablinse während der Handhabung des Endoskops sowie während dessen Reinigung verhindert.

Durch das Vorsehen des Luftspalts gibt es zudem keinen direkten Kontakt zwischen der Außenseite der Stablinse und der Innenseite des Rohrs, so daß auch bei Schüttelbeanspruchung, etwa bei Reinigung, kein Abrieb erzeugt wird, der die Abbildungsqualität beeinträchtigen würde.

Somit wird die erfindungsgemäße Aufgabe vollkommen gelöst.

Die genaue Bemessung des zwischen der Außenseite der Stablinse und der Innenseite des Rohrs vorgesehenen radialen Abstands oder Luftspalts kann je nach der gewünschten Anwendung unterschiedlich sein. Der Fachmann kann die Bemessung des Abstands unter Berücksichtigung der Länge der Stablinse bzw. mehrerer Stablinsen, die innerhalb eines Rohres auch unterschiedliche Längen aufweisen können, des Materials, aus dem das Rohr besteht, sowie aus Form und Größe der Klebestellen sowie dem jeweils verwendeten Klebstoff vornehmen.

In einer bevorzugten Ausgestaltung wird der radiale Abstand zwischen der Außenseite der Stablinse und der Innenseite des Rohrs so ausgewählt, daß er im Bereich von etwa 0,05 bis 0,3 mm, vorzugsweise im Bereich von etwa 0,1 mm liegt.

Bei einem derartigen Abstand ist sowohl das Einführen der Stablinse in das Rohr sowie das Bewerkstelligen der Klebeverbindung problemlos möglich, da genügend Spielraum zum Einschieben vorgesehen ist, ohne daß die Klebestellen dabei einen zu großen radialen Abstand überbrücken müssen, wodurch die Klebeverbindung instabil werden könnte.

In einer weiteren Ausgestaltung wird das Positionieren mit zumindest drei um 120° umfänglich versetzt angeordneten Zentrierelementen je Zentrierhilfe durchgeführt.

Diese Maßnahme hat den Vorteil, daß die Stablinse besonders sicher abgestützt wird, und somit ein Verschieben oder Verkippen der Stablinse nicht mehr möglich ist.

Es versteht sich, daß je nach dem Außendurchmesser der Stablinse auch mehr als drei Zentrierelemente vorgesehen werden können, um die Linse abzustützen.

In einer weiteren Ausgestaltung läßt man die Zentrierelemente nach dem Bewerkstelligen der Klebeverbindung in dem Zusammenbau.

Dazu können die Zentrierelemente als Abstandshalter vorgesehen werden, die in Form von mehr oder weniger großen Abstandsstükken oder sich radial oder axial erstreckenden Streifen vorliegen. Sie können aus einem beliebigen Material, bspw. aus Kunststoff, bestehen.

Hierbei ist vorteilhaft, daß kein Entfernen der Zentrierelemente nach dem Montieren notwendig ist, was das erfindungsgemäße Verfahren erheblich vereinfacht. In dem Fall, wenn die Abstandshalter aus einem elastischen Material bestehen, sorgen sie ferner für einen zusätzlichen Schutz der Linse gegen Krafteinwirkung von außen.

In einer weiteren Ausgestaltung werden zum Positionieren längliche, insbesondere zylindrische Metall- oder Kunststoffteile ausgewählt, die sich axial zwischen der Innenseite des Rohrs und der Außenseite der Stablinse erstreckend angelegt werden, und man entfernt diese nach dem Bewerkstelligen der Klebeverbindung wieder oder läßt sie in dem Zusammenbau.

In einer bevorzugten Ausgestaltung werden zum Positionieren sich radial erstreckende Zentrierstifte eingesetzt, die durch Öffnungen in der Wand des Rohrs hindurchtreten und die nach dem Bewerkstelligen der Klebeverbindung entfernt werden.

Bei dieser Maßnahme ist vorteilhaft, daß das Positionieren und Zentrieren der Stablinse besonders präzise durchgeführt werden kann, da auch nach vollständigem Einführen der Stablinse in das Innere des Rohrs durch die Öffnungen in der Wand des Rohrs direkt auf die Außenseite der Stablinse zugegriffen werden kann. So kann die Stablinse zunächst in ihre genaue axiale Position gebracht werden, und anschließend kann deren radiale bzw. zentrale Lage eingestellt werden. Ein weiterer Vorteil dieser Maßnahme besteht darin, daß die Zentrierelemente von außen gehandhabt werden können, was eine erhebliche Erleichterung des Zentriervorgangs mit sich bringt.

In einer Ausgestaltung ist an jeder Klebestelle zumindest eine Öffnung in der Wand des Rohrs vorgesehen, durch die der Klebstoff zum Bewerkstelligen der Klebeverbindung zwischen dem Rohr und der Stablinse gegeben wird.

Diese Maßnahme hat den erheblichen Vorteil, daß der Klebstoff erst dann zugegeben werden muß, wenn der Zentriervorgang beendet und die Stablinse präzise im Inneren des Rohrs positioniert worden ist. Hierbei ist ferner von Vorteil, daß der Klebstoff durch die Öffnung an einer definierten Stelle aufgetragen werden kann, so daß er sich nicht durch nachfolgendes weiteres axiales oder radiales Verschieben der Stablinse an ungewünschten Stellen verteilt und dann die Toleranz des Zusammenbaus aus dem Rohr und der Stablinse gegenüber Biegebelastungen beeinträchtigt.

In einer weiteren Ausgestaltung werden drei gleichmäßig um den Umfang des Rohrs verteilte Öffnungen vorgesehen, durch die der Klebstoff zum Bewerkstelligen der Klebeverbindung zwischen dem Rohr und der Stablinse und in Form von Klebepunkten aufgetragen wird.

Diese Maßnahme hat den wesentlichen Vorteil, daß die Stablinse derart im Inneren des Rohrs fixiert wird, daß ein axiales oder ein radiales Verschieben der Linse sicher verhindert wird. Dabei bleibt aufgrund der Elastizität des punktuell aufgetragenen Klebstoffs die Flexibilität des Zusammenbaus gewährleistet.

Bei dieser Maßnahme ist ferner vorteilhaft, daß ein Druckausgleich zwischen den beiden axialen Enden des Rohrs möglich wird. Bei Luftdruckdifferenzen zwischen den beiden Enden können nämlich Längskräfte auftreten, die die Klebestellen belasten.

In einer weiteren Ausgestaltung wird der Klebstoff zum Bewerkstelligen der Klebeverbindung zwischen dem Rohr und der Stablinse durch die Öffnung in Form eines Kleberings aufgetragen, der den Umfang der Stablinse im Bereich der Klebestelle umschließt.

Diese Maßnahme hat den Vorteil, daß die Außenseite der Stablinse im Bereich der Klebestelle umfänglich mit der Innenseite des Rohrs verklebt und damit besonders sicher fixiert wird. Ein Verkippen der Stablinse im Inneren des Rohrs ist in jeder Richtung vollständig ausgeschlossen.

In einer weiteren Ausgestaltung treten die Zentrierstifte durch diejenigen Öffnungen hindurch, durch die auch der Klebstoff aufgetragen wird, wobei die Außenseite der Zentrierstifte aus einem den Klebstoff abweisenden Material bestehen oder mit einem solchen beschichtet sind, und diese können nach dem Bewerkstelligen der Klebeverbindung aus dem Zusammenbau wieder entfernt werden.

Diese Maßnahme hat den Vorteil, daß zum Auftragen des Klebstoffs keine zusätzlichen Löcher in der Wand des Rohrs vorgesehen werden müssen. So können zunächst die Zentrierstifte durch die Öffnungen hindurchgeführt werden, um die Stablinse zu zentrieren, anschließend wird durch dieselben Öffnungen der Klebstoff eingeführt. Der Klebstoff kann danach aushärten, und erst zu diesem Zeitpunkt werden die Zentrierstifte vom Zusammenbau abgezogen.

In einer weiteren Ausgestaltung der Erfindung werden Zentrierstifte eingesetzt, die kegelige Spitzen aufweisen.

Bei dieser Maßnahme ist vorteilhaft, daß ein Herausziehen der Zentrierstifte aus dem ausgehärteten Klebstoff besonders leicht durchzuführen ist.

In einer alternativen Ausgestaltung des erfindungsgemäßen Verfahrens werden als Zentrierelemente Klebepunkte entweder auf der Außenseite der Stablinse oder auf der Innenseite des Rohrs aufgetragen, die sich durch Aktivieren ausdehnen oder auch, durch Oberflächenkräfte, Kugelform anstreben.

Diese Ausführungsform hat den erheblichen Vorteil, daß die Zentrierelemente hier eine Doppelfunktion einerseits zum Zentrieren und andererseits zum Bewerkstelligen der Klebeverbindung haben. Dies erleichtert das erfindungsgemäße Verfahren zusätzlich, da hier weder Öffnungen zum Einführen der Zentrierelemente noch zum Auftragen des Klebstoffs in der Wand des Rohrs vorgesehen sein müssen.

Die Klebepunkte können an gewünschten Positionen auf der Außenseite der Stablinse oder auf der Innenseite des Rohrs aufgebracht werden, wobei der aufgebrachte Klebstoff eine radiale Ausdehnung aufweist, die derart bemessen ist, daß ein Großteil des Luftspalts zwischen der Außenseite der Stablinse und der Innenseite des Rohrs überbrückt wird, wenn die Stablinse in das Rohr eingeführt wird. Dabei ist jedoch noch genügend Spielraum vorhanden, damit ein problemloses Einführen der Stablinse in das Rohr möglich ist. Beim Einschieben der Stablinse in das Rohr wird die Stablinse dann aufgrund der Erhabenheit der Klebepunkte "automatisch" zentriert. Der zwischen dem Klebepunkt und z.B. der Innenseite des Rohrs verbleibende Spielraum wird erst durch das Aktivieren des Klebstoffs aufgrund seiner dabei erfolgenden Ausdehnung erfüllt, so daß letztendlich eine feste Verbindung zwischen der Außenseite der Stablinse mit der Innenseite des Rohrs unter Vermittlung des Klebstoffpunkts hergestellt wird.

Vorzugsweise erfolgt das Aktivieren des Klebstoffs durch Wärmebehandlung.

Eine derartige Wärmebehandlung ist besonders einfach durchzuführen. Die hierfür notwendige Temperatur liegt über der notwendigen Temperatur zum Sterilisieren des Endoskops.

Je nach dem verwendeten aktivierbaren Klebstoff kann das Aktivieren jedoch auch durch UV-Bestrahlung oder weitere geeignete Maßnahmen erfolgen.

In einer bevorzugten Ausgestaltung werden die Klebepunkte von einem Material umgeben, das als Barriere dient.

Dieses Material kann die Klebepunkte bspw. ringförmig umgeben, wobei auch ein Klebstoff abweisendes Material eingesetzt werden kann.

Diese Maßnahme hat den Vorteil, daß die Ausdehnung des Klebstoffs nach dessen Auftragen seitlich begrenzt ist und im wesentlichen nur in radialer Richtung möglich ist.

In einer weiteren Ausgestaltung der Erfindung sind an der Außenseite der Stablinse elastische Zentrierelemente angebracht, die mit der Innenseite des Rohres verklebt werden.

Diese Maßnahme hat den Vorteil, daß die Zentrierelemente schon selbst als Bestandteil der elastischen Klebeverbindung ausgebildet sind. Es werden dann die elastischen Zentrierelemente mit der Innenseite des Rohres verklebt und dadurch die insgesamt elastische Klebeverbindung zwischen Innenseite des Rohres und Außenseite der Stablinse bewerkstelligt.

In einer weiteren Ausgestaltung der Erfindung wird, bei einer als Knochenlinse ausgebildeten Stablinse mit radialen Verdikkungen die Klebeverbindung benachbart zur Verdickung angebracht.

Bei manchen Endoskopen werden sogenannte Knochenlinsen eingesetzt, die meist mit endseitigen radialen Verdickungen versehen sind. Im Bereich der Verdickung ist das Radialmaß der Linse größer als zwischen den Verdickungen. Wird nun die elastische Klebeverbindung in der Nähe der Verdickung angebracht, können dennoch erhebliche Verbiegungen des Rohres zwischen diesen beiden Klebestellen, also zwischen den radialen Verdickungen stattfinden, ohne daß die Knochenlinse beeinträchtigt wird, so daß die Erfindung auch bei solchen Konstruktionen unter den vorgenannten Bedingungen einsetzbar ist.

Es versteht sich, daß die vorstehend genannten und die nachstehend noch zu erläuternden Merkmalen nicht nur in den angegebenen Kombinationen, sondern auch in anderen Kombinationen oder in Alleinstellung verwendbar sind, ohne den Rahmen der vorliegenden Erfindung zu verlassen.

Weitere Vorteile und Ausgestaltungen der Erfindung werden nachstehend unter Bezugnahme auf die beigefügten Zeichnungen erläutert. Es zeigen:
- Fig. 1: eine teilweise aufgebrochene Seitenansicht eines erfindungsgemäßen Endoskops;
- Fig. 2: einen Teillängsschnitt eines Endoskops gemäß Fig. 1, und zwar im Bereich eines Zusammenbaus aus einem Rohr und einer Stablinse;
- Fig. 3: einen Querschnitt längs der Linie III-III in Fig. 2;
- Fig. 4: einen Ausschnitt entsprechend Fig. 2, wobei das Rohr aufgrund von Belastungen gebogen ist;
- Fig. 5: einen Ausschnitt aus einer weiteren Ausführungsform eines erfindungsgemäßen Zusammenbaus im Längsschnitt;
- Fig. 6: einen Querschnitt längs der Linie IV-IV;
- Fig. 7 bis 10: Querschnitte durch weitere Ausführungsformen eines erfindungsgemäßen Zusammenbaus;
- Fig. 11: einen Ausschnitt aus einer alternativen Ausführungsform eines erfindungsgemäßen Zusammenbaus im Längsschnitt;
- Fig. 12: einen Schnitt längs der Linie XII-XII von Fig. 11;
- Fig. 13: einen vergrößerten Ausschnitt der Ausführung von Fig. 11, und zwar im Bereich einer Klebestelle vor Aktivierung des Klebstoffs;
- Fig. 14: den Ausschnitt von Fig. 13 nach Aktivierung des Klebstoffs;
- Fig. 15: einen der Darstellung von Fig. 13 entsprechenden Ausschnitt einer weiteren Ausführungsform vor Aktivierung des Klebstoffs;
- Fig. 16: den Ausschnitt von Fig. 15 nach Aktivieren des Klebstoffs;
- Fig. 17: einen der Darstellung von Fig. 13 vergleichbaren Ausschnitt einer weiteren Ausführungsform mit elastischen Zentrierelementen;
- Fig. 18: die Darstellung von Fig. 17 nach Bewerkstelligung der Klebeverbindung; und
- Fig. 19: einen teilweisen Längsschnitt eines Endoskopes, in den eine Knochenlinse montiert ist.

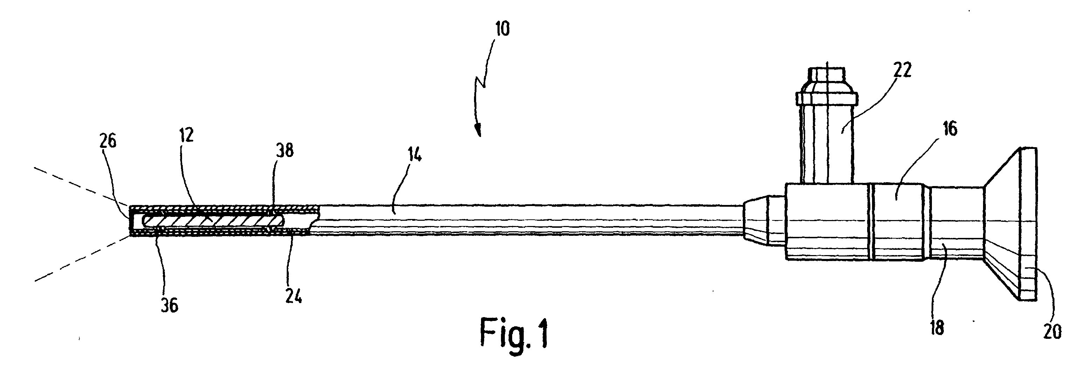

Bei einem in Fig. 1 dargestellten Endoskop, das in seiner Gesamtheit mit der Bezugsziffer 10 bezeichnet ist, wurde das erfindungsgemäße Verfahren zum Montieren einer Stablinse 12 im Inneren des Endoskops 10 verwendet.

Das Endoskop 10 weist einen langerstreckten rohrförmigen Schaft 14 auf, an dessen proximalem Ende ein Gehäuse 16 angeordnet ist. In dem Gehäuse 16 ist ein Okular 18 mit einer Okularmuschel 20 vorgesehen.

In dem Rohrschaft 14 des Endoskops 10 ist ein optisches System aufgenommen, zu dem auch die Stablinse 12 gehört. Zusammen mit weiteren, hier nicht dargestellten Linsen, Blenden, Filtern und dergleichen dient das optische System dazu, ein am distalen Ende erfaßbares Bild zum proximalen Ende zu bringen. Dazu ist ferner in dem Rohrschaft 14 ein hier nicht näher bezeichnetes Lichtleitsystem in Form von Glasfasern angeordnet, die über einen seitlich von dem Gehäuse 16 abführenden Ansatz 22 mit einer hier nicht dargestellten Lichtquelle verbunden werden.

Im Inneren des Rohrschafts 14 ist ein inneres Rohr 24 angeordnet, innerhalb dessen die Stablinse 12 und die nicht dargestellten weiteren Bestandteile des optischen Systems angeordnet werden.

Ein Fenster 26 am distalen Ende des Rohrs 24 bildet einen dichten Abschluß der distalen Seite des Rohrs 24, über das die Bildinformation in das optische System eintritt. Ist das Rohr 24 aufgrund der unten genannten Klebe- oder Justieröffnungen nicht hermetisch abdichtbar, so kann ein hier nicht dargestelltes Zwischenrohr vorgesehen sein, das dann anstelle des Rohrs 24 durch das Fenster 26 dicht abgeschlossen wird.

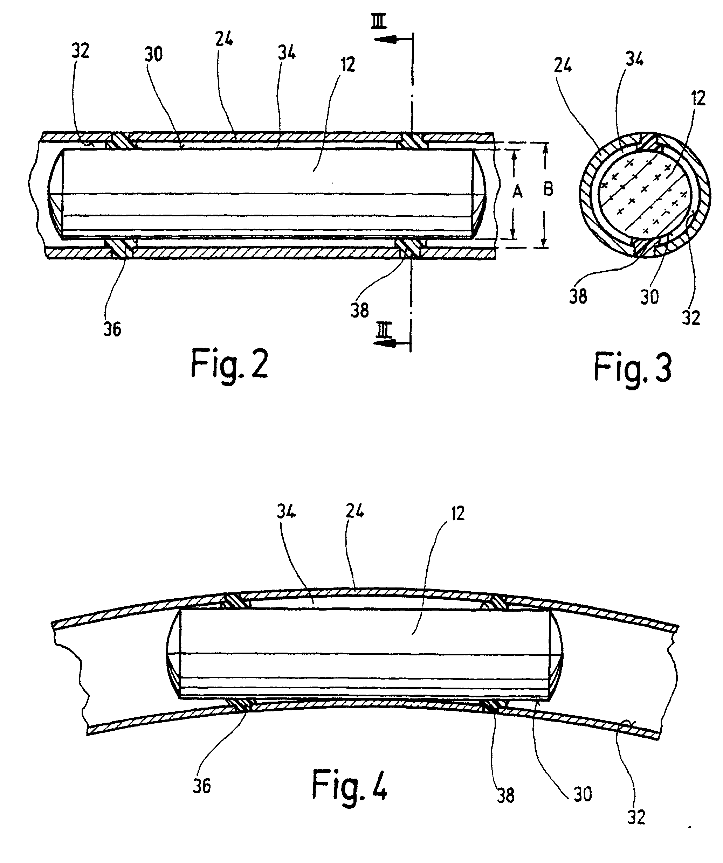

In den Fig. 2 bis 4 ist ein Ausschnitt des Rohrs 24 mit der in dessen Innerem angeordneten Stablinse 12 vergrößert dargestellt.

In diesen Darstellungen ist gut ersichtlich, daß der Außendurchmesser A der Stablinse 12 kleiner als der lichte Innendurchmesser B des Rohrs 24 ist, so daß zwischen der Außenseite 30 der Stablinse 12 und der Innenseite 32 des Rohrs 24 ein radialer Abstand oder Luftspalt 34 vorhanden ist. Die Außenseite 30 der Stablinse 12 hat keinen direkten Kontakt zu der Innenseite 32 des Rohrs 24, sondern ist über Klebepunkte 36 und 38 zentrisch innerhalb des Rohrs 24 fixiert.

Es versteht sich, daß die Größenverhältnisse hier nicht maßstabsgetreu abgebildet sind. Vor allem die Darstellung des Luftspalts 34 und der Klebepunkte 36 und 38 sind aus Anschaulichkeitsgründen stark vergrößert dargestellt.

In dem in den Fig. 2 bis 4 gezeigten Ausführungsbeispiel ist die Stablinse 12 über insgesamt vier Klebepunkte 36 befestigt. Jeweils zwei Klebepunkte 36 sind gleichmäßig um den Umfang der Stablinse 12 verteilt, also um 180° versetzt angeordnet. Außerdem sind die beiden Klebepunkte 36 und 38 axial voneinander beabstandet.

Durch die Klebepunkte 36 und 38 ist die Stablinse 12 lagegerecht im Inneren des Rohrs 24 fixiert, ohne daß ein axiales Verschieben oder Verkippen der Stablinse 12 möglich ist.

Die durch das Zusammenwirken des Luftspalts 34 mit den Klebepunktes 36 und 38 hervorgerufene Flexibilität des Zusammenbaus gegenüber Biegebelastungen geht aus Fig. 4 hervor. Dort ist gezeigt, daß ein bogenförmiges Abbiegen des Rohrs 24 in einem gewissen Ausmaß möglich ist, ohne daß die Stablinse 12 einer hohen Biegebelastung ausgesetzt wird. Aufgrund des Luftspalts 34 und der Elastizität der Klebepunkte 36 und 38 ist nämlich ein Abbiegen des Rohrs 24 über die gesamte Länge der Stablinse 12 möglich, wobei die Klebepunkte 36 und 38 sich in einem gewissen Maß deformieren können.

Erst ab dem in Fig. 4 gezeigten Ausmaß des Verbiegens des Rohrs 24, nämlich dann, wenn die Innenseite 32 des Rohrs 24 die Außenseite 30 der Stablinse 12 berührt, wird eine Biegebelastung auch auf die Stablinse 12 ausgeübt.

Es versteht sich, daß statt der Klebepunkte 36 und 38 auch Kleberinge vorgesehen werden können, die die Stablinse 12 im Bereich der Klebestellen umfänglich umschließen.

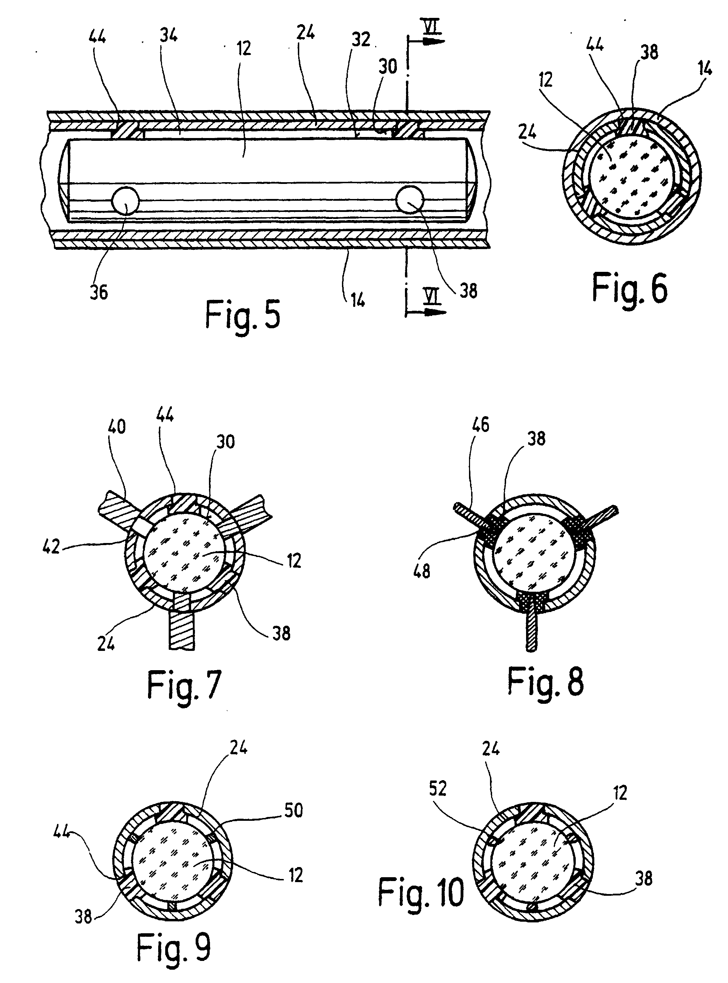

Eine weitere Ausführungsform des Zusammenbaus aus dem Rohr 24 und der Stablinse 12, anhand derer das erfindungsgemäße Verfahren zum Montieren dieser Bauteile erläutert werden soll, ist in den Fig. 5 bis 10 gezeigt.

Hierbei ist die Stablinse 12 an zwei axial voneinander beabstandeten Stellen durch jeweils drei um den Umfang der Stablinse verteilte Klebepunkte 36 und 38 im Inneren des Rohrs 24 verbunden. Jeweils drei umfänglich verteilte Klebepunkte 36 bzw. 38 erlauben eine besonders sichere Fixierung der Stablinse im Inneren des Rohrs.

Zum Montieren der Stablinse 12 wird diese zunächst in das Rohr 24 eingeschoben, was aufgrund des Luftspalts 34, der im Bereich von etwa 0,1 mm liegt, problemlos möglich ist. Zum Positionieren der Stablinse 12 zentrisch im Inneren des Rohrs 24, d.h. um einen definierten Abstand der Außenseite 30 der Stablinse zu der Innenseite 32 des Rohrs herzustellen, sind aus mehreren Zentrierelementen bestehende Zentrierhilfen vorgesehen, deren verschiedene Ausführungsformen in den Fig. 7 bis 10 dargestellt sind.

Bei der in Fig. 7 dargestellten Ausführungsform werden Zentrierelemente in Form von Zentrierstiften 40 durch Öffnungen 42 der Wand des Rohrs 24 eingeführt. Die drei gleichmäßig um den umfang der Stablinse 12 verteilten Zentrierstifte 40, die durch die Öffnungen 42 in der Außenseite 30 der Stablinse 12 angreifen, bilden eine Zentrierhilfe. Um ein präzises Positionieren der Stablinse 12 zu erlauben, sind zwei axial voneinander beabstandete Zentrierhilfen vorgesehen.

Nachdem die Stablinse 12 durch die Zentrierhilfen in dem gewünschten Abstand zu der Innenseite 32 des Rohrs 24 positioniert wurde, erfolgt das Bewerkstelligen der Klebeverbindung zwischen der Außenseite 30 der Stablinse 12 und der Innenseite 32 des Rohrs 24. Dazu wird durch weitere Öffnungen 44, die ebenfalls gleichmäßig um den Umfang der Stablinse 12 verteilt sind, Klebstoff in Form von Klebepunkten 36, 38 aufgetragen. Jeder Klebepunkt 36 bzw. 38 füllt die Öffnung 44 in der Wand des Rohrs 24 aus und überbrückt in einem begrenzten Bereich den Luftspalt 34 zwischen der Außenseite 30 der Stablinse 12 und der Innenseite 32 des Rohrs.

Wenn der Klebstoff ausgehärtet ist, können die Zentrierstifte 40 aus den Öffnungen 42 der Wand des Rohrs 24 abgezogen werden.

Dieser Zusammenbau kann danach in den in den Fig. 5 und 6 gezeigten Rohrschaft 14 des Endoskops 10 eingeschoben werden.

Eine weitere Ausführungsform der Zentrierhilfe ist in Fig. 8 abgebildet.

Hier gezeigte Zentrierstifte 46 greifen durch die zum Auftragen des Klebstoffs vorgesehenen Öffnungen 44 an der Linse an. Nachdem die Stablinse 12 mit Hilfe der Zentrierstifte 46 zentrisch im Inneren des Rohrs 24 positioniert wurde, wird durch die Öffnungen 44 Klebstoff in Form von Klebepunkten aufgetragen. Dies ist problemlos möglich, da die Zentrierstifte 46 in dem in Fig. 8 gezeigten Ausführungsbeispiel dünner als die Öffnungen 44 sind, sie weisen außerdem kegelförmige Spitzen 48 auf. Damit die Zentrierstifte 46 nach dem Aushärten des Klebstoffs wieder einfach entfernt werden können, können diese aus einem klebstoffabweisenden Material bestehen oder sind mit einem solchen Material beschichtet.

Die in den Fig. 9 und 10 vorgesehenen Zentrierhilfen müssen nach der Montage nicht unbedingt aus dem Zusammenbau entfernt werden.

In Fig. 9 liegen die Zentrierhilfen in Form von drei umfänglich um die Stablinse 12 verteilten Abstandshaltern 50 vor, die entweder als Abstandsstücke oder als sich axial erstreckende Streifen, die bspw. aus Kunststoff bestehen, vorgesehen sein können. Die Abstandshalter 50 können gleichzeitig mit der Linse in das Rohr 24 eingeschoben werden. Ein Entfernen der Abstandshalter 50 nach Anbringen der Klebepunkte 38 ist in dieser Ausführungsform ebenso wenig notwendig wie das Vorsehen von Öffnungen 44 für die Zentrierhilfen in der Wand des Rohres 24.

Die in Fig. 10 dargestellte Zentrierhilfe liegt in Form von sich axial erstreckenden länglichen zylindrischen Körpern 52 vor, die um den Umfang der Stablinse 12 gleichmäßig verteilt sind. Die zylindrischen Körper 52 können nach Auftragen und Aushärten der Klebepunkte 38 entweder in dem Zusammenbau verbleiben oder aber herausgezogen werden.

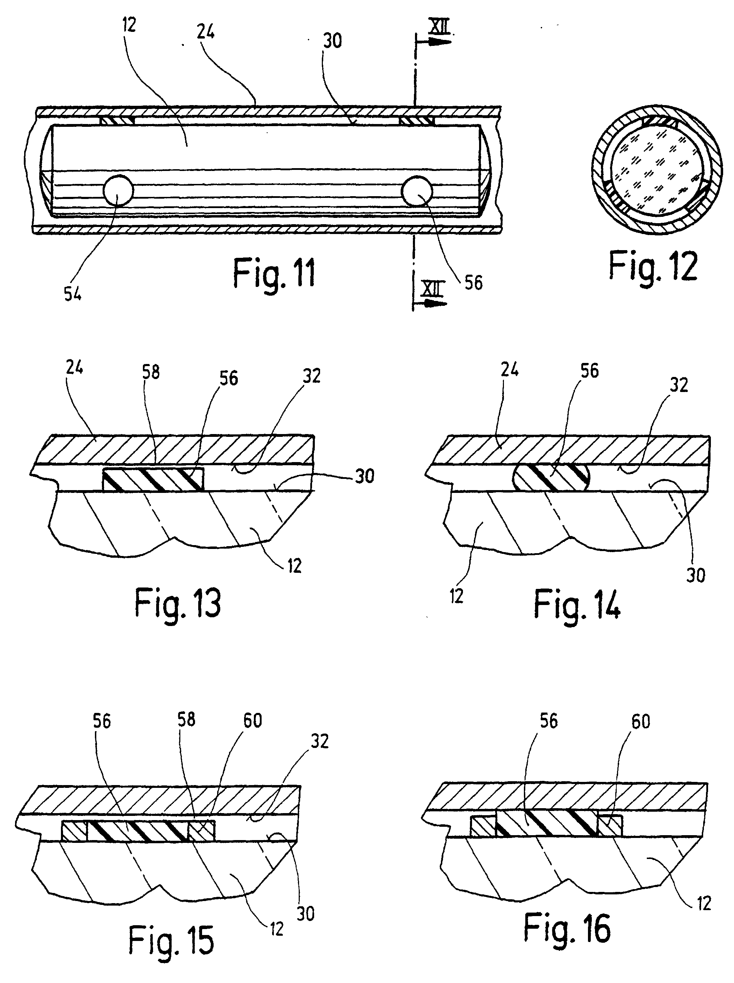

Eine alternative Ausführungsform des erfindungsgemäßen Montageverfahrens ist in den Fig. 11 bis 16 gezeigt. Bei dieser Ausgestaltung ist es nicht notwendig, Öffnungen in der Wand des Rohrs 24 vorzusehen. Das Zentrieren der Stablinse 12 erfolgt bei dieser Ausgestaltung nämlich durch Klebepunkte 54 und 56 selbst. Dazu wird ein Klebstoff verwendet, der sich durch Aktivierung ausdehnen oder auch Kugelform annehmen kann.

So können Klebepunkte 54 und 56 zunächst auf die Außenseite 30 der Stablinse 12 aufgetragen werden, und zwar noch bevor die Stablinse 12 in das Rohr 24 eingeschoben wurde.

Die Verteilung der Klebepunkte 54 und 56 ist beliebig, muß jedoch an mindestens zwei axial zueinander beabstandeten Stellen erfolgen. Um eine möglichst sichere Fixierung der Stablinse zu erlauben, werden vorzugsweise an zwei axial beabstandeten Stellen drei um den Umfang der Stablinse 12 verteilte Klebepunkte 54, 56 angebracht.

Nach Auftragen der Klebepunkte 54, 56 auf die Außenseite 30 der Stablinse 12 wird die Stablinse 12 in das Rohr 24 eingeführt.

Aus Fig. 13 ist ersichtlich, daß der Klebepunkt 56 sich radial von der Oberfläche 30 der Stablinse 12 erhebt, wobei jedoch noch ein geringer Spalt 58 zwischen dem Klebepunkt 56 und der Innenseite 32 des Rohrs 24 frei bleibt, um das Einschieben der Stablinse 12 zu ermöglichen.

Danach wird der Klebstoff bspw. durch Wärmebehandlung aktiviert, wodurch er sich ausdehnt oder verformt, bspw. aufgrund von Oberflächenkräften Kugelform anstrebt, siehe Fig. 14. Dadurch füllt sich der Spalt 58 mit Klebstoff im Bereich des Klebepunktes 56, der Klebepunkt 56 verklebt die Innenseite 32 des Rohrs 24 mit der Außenseite 30 der Stablinse 12. und stellt somit eine feste Verbindung her.

Um die Ausdehnung der Klebepunkte zu begrenzen, können diese von einer Barriere bspw. in Form eines Abstandsrings 60 umgeben werden, wie in den Fig. 15 und 16 gezeigt ist. Durch den Abstandsring 60 wird verhindert, daß sich der Klebstoff bei der Aktivierung über größere Flächen der Außenseite 30 der Stablinse 12 seitlich verteilt, sondern sich im wesentlichen in radialer Richtung ausdehnen kann.

Wie in Fig. 16 gezeigt ist, bewerkstelligt ein Klebepunkt 56 nach Aktivierung des Klebstoffs die Verbindung zwischen der Innenseite 32 des Rohrs 24 und der Außenseite 30 der Stablinse 12.

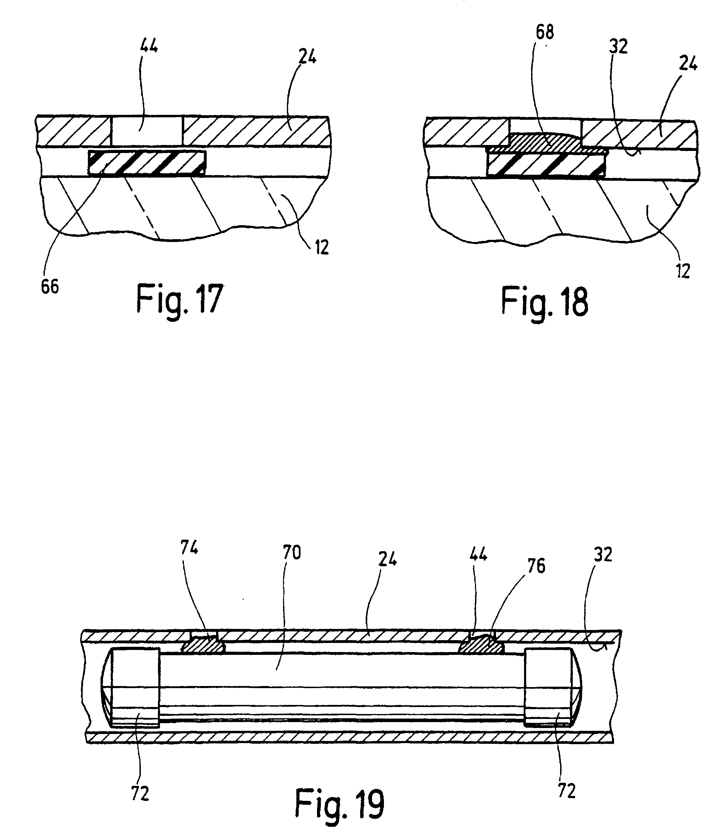

Bei dem in den Fig. 17 und 18 dargestellten Ausführungsbeispiel sind auf der Außenseite der Stablinse 12 elastische Zentrierelemente 66 aufgebracht, die entweder punktförmig, wie zuvor beschrieben, länglich oder ringförmig ausgebildet sein können. Diese elastischen Zentrierelemente dienen zur lagegerechten Positionierung der Linse 12 im Rohr 24. Zur Bewerkstelligung der Klebeverbindung wird über eine Öffnung 44 im Rohr 24 der Klebstoff 68 eingebracht, der dann eine Verbindung zwischen dem elastischen Zentrierelement 66 und der Innenseite 32 des Rohres 24 bildet. Auch dadurch resultiert dann insgesamt eine elastische Klebeverbindung zwischen der Außenseite der Stablinse 12 und der Innenseite 32 des Rohres 24.

In Fig. 19 ist eine Ausführung einer Stablinse als Knochenlinse 70 dargestellt, die mit endseitigen Verdickungen 72 versehen ist. Die Knochenlinse 70 ist, wie zum Beispiel im Zusammenhang mit Fig. 2 beschrieben, über Klebepunkte 74 und 76 mit dem Rohr 24 verbunden, somit also die Außenseite der Knochenlinse 70 über eine elastische Klebeverbindung mit der Innenseite 32 des Rohres 24 verbunden. Betrachtet man Fig. 4, so ist daraus zu erkennen, daß die wesentlichen Biegungen zwischen den Klebepunkten stattfinden. Liegen, wie in Fig. 19 dargestellt, die Klebepunkte 74 und 76 benachbart zu den Verdickungen 70 und 72, so kann zwischen den Klebepunkten 74 und 76 bzw. dann entsprechend zwischen den Verdickungen 72 ebenfalls ein relativ starkes Verbiegen des Rohres 24 stattfinden, wie das in Fig. 4 angedeutet ist.

## Patentansprüche

1. Verfahren zum Montieren zumindest einer Stablinse (12, 70) im Inneren eines zu einem Endoskop (10) gehörenden Rohrs (24) durch Fixieren der Stablinse (12, 70) im Inneren des Rohrs (24) an zumindest zwei axial voneinander beabstandeten Stellen, mit folgenden Schritten:
- Auswählen des Außendurchmessers (A) der Stablinse (12, 70) derart, daß nach Einbringen der Stablinse (12, 70) in das Rohr (24) zwischen der Außenseite (30) der Stablinse (12, 70) und der Innenseite (32) des Rohrs (24) umfänglich ein Luftspalt (34) entsteht, und
- Positionieren der Außenseite (30) der Stablinse (12, 70) in einem definierten radialen Abstand zu der Innenseite (32) des Rohrs (24) durch zumindest zwei Zentrierhilfen, die an zumindest zwei axial voneinander beabstandeten Stellen der Außenseite (30) der Stablinse (12, 70) angreifen, wobei jede Zentrierhilfe Zentrierelemente (66) aufweist, welche gleichmäßig um den Umfang der Stablinse (12, 70) verteilt sind,
**gekennzeichnet durch**
ein Bewerkstelligen von direkten elastischen Klebeverbindung zwischen der Außenseite (30) der Stablinse (12, 70) und der Innenseite (32) des Rohrs (24) an den axial voneinander beabstandeten Stellen.

2. Verfahren nach Anspruch 1, **dadurch gekennzeichnet, daß der** radiale Abstand zwischen der Außenseite der Stablinse (12, 70) und der Innenseite (32) des Rohrs (24) so ausgewählt wird, daß er im Bereich von etwa 0,05 bis 0,3 mm, vorzugsweise im Bereich von etwa 0,1 mm liegt.

3. Verfahren nach Anspruch 1 oder 2, **dadurch gekennzeichnet, daß** das Positionieren mit drei um ca. 120° umfänglich versetzt angeordneten Zentrierelementen je Zentrierhilfe durchgeführt wird.

4. Verfahren nach einem der Ansprüche 1 bis 3, **dadurch gekennzeichnet, daß** man die Zentrierelemente (66) nach dem Bewerkstelligen der Klebeverbindung in dem Zusammenbau aus Rohr (24) und Stablinse (12) beläßt.

5. Verfahren nach einem der Ansprüche 1 bis 3, **dadurch gekennzeichnet, daß** zum Positionieren längliche, insbesondere zylindrische Körper (52) ausgewählt werden, die sich axial zwischen der Innenseite (32) des Rohrs (24) und der Außenseite (30) der Stablinse (12) erstreckend angelegt werden und daß man diese nach dem Bewerkstelligen der Klebeverbindung wieder entfernt oder in dem Zusammenbau läßt.

6. Verfahren nach einem der Ansprüche 1 bis 3, **dadurch gekennzeichnet, daß** zum Positionieren sich radial erstrekkende Zentrierstifte (40; 46) eingesetzt werden, die durch Öffnungen (42) in der Wand des Rohres (24) hindurchtreten und die nach dem Bewerkstelligen der Klebeverbindung entfernt werden.

7. Verfahren nach einem der Ansprüche 1 bis 6, **dadurch gekennzeichnet, daß** an jeder Klebestelle zumindest eine Öffnung (44) in der Wand des Rohres (24) vorgesehen wird, durch die der Klebstoff zum Bewerkstelligen der Klebeverbindung zwischen dem Rohr (24) und der Stablinse (12) eingebracht wird.

8. Verfahren nach Anspruch 7, **dadurch gekennzeichnet, daß** drei gleichmäßig um den Umfang des Rohres (24) verteilte Öffnungen (44) vorgesehen werden, durch die der Klebstoff zum Bewerkstelligen der Klebeverbindung zwischen dem Rohr (24) und der Stablinse (12) in Form von Klebepunkten (36, 38) eingebracht wird.

9. Verfahren nach Anspruch 7, **dadurch gekennzeichnet, daß** der Klebstoff zum Bewerkstelligen der Klebeverbindung zwischen dem Rohr (24) und der Stablinse (12) durch die Öffnung (44) in Form eines Kleberings aufgetragen wird, der den Umfang der Stablinse (12) im Bereich der Klebestelle umschließt.

10. Verfahren nach einem der Ansprüche 7 bis 9, **dadurch gekennzeichnet, daß** die Zentrierstifte (46) durch diejenigen Öffnungen (44) hindurchtreten, durch die auch der Klebstoff aufgetragen wird, daß die Außenseite der Zentrierstifte aus einem den Klebstoff abweisenden Material bestehen oder mit einem solchen beschichtet sind, und daß diese nach dem Bewerkstelligen der Klebeverbindung aus dem Zusammenbau wieder entfernt werden.

11. Verfahren nach Anspruch 10, **dadurch gekennzeichnet, daß** Zentrierstifte (46) eingesetzt werden, die kegelige Spitzen (48) aufweisen.

12. Verfahren nach einem der Ansprüche 1 bis 3, **dadurch gekennzeichnet, daß** als Zentrierelemente Klebepunkte (54, 56) auf der Außenseite (30) der Stablinse (12) oder auf der Innenseite (32) des Rohrs (24) aufgetragen werden, die sich durch Aktivieren des Klebstoffs ausdehnen oder verformen.

13. Verfahren nach Anspruch 12, **dadurch gekennzeichnet, daß** das Aktivieren des Klebstoffs durch Wärmebehandlung erfolgt.

14. Verfahren nach Anspruch 12 oder 13, **dadurch gekennzeichnet, daß** die Klebepunkte (54, 56) aus Klebstoff von einem Material oder einer Oberfläche umgeben werden, das bzw. die als Barriere für die Ausdehnung des Klebstoffs dient.

15. Verfahren nach einem der Ansprüche 1 bis 4, **dadurch gekennzeichnet, daß** an der Außenseite der Stablinse (12) elastische Zentrierelemente (66) angebracht werden, die mit der Innenseite (32) des Rohres (24) verklebt werden.

16. Verfahren nach einem der Ansprüche 1 bis 15, **dadurch gekennzeichnet, daß** bei einer als Knochenlinse (70) ausgebildeten Stablinse mit radialen Verdickungen (72) die Klebeverbindung (74, 76) benachbart zur Verdickung (70) angebracht wird.

17. Endoskop (10) mit einem Rohr (24), in dessen Innerem zumindest eine Stablinse (12) an zumindest zwei axial voneinander beabstandeten Stellen fixiert ist, wobei die Stablinse (12) einen Außendurchmesser (A) aufweist, der derart bemessen ist, daß nach Einbringen der Stablinse (12) in das Rohr (24) zwischen der Außenseite (30) der Stablinse (12) und der Innenseite (32) des Rohrs (24) ein Luftspalt (34) vorhanden ist, und wobei die Außenseite (30) der Stablinse (12) in einem definierten radialen Abstand zu der Innenseite (32) des Rohrs (24) angeordnet ist, **dadurch gekennzeichnet, daß** die Außenseite (30) der Stablinse (12) durch direkte elastische Klebestellen mittels Klebstoff an der Innenseite (32) des Rohrs (24) an den voneinander beabstandeten Stellen fixiert ist, wobei der Raum zwischen den axial von ein ander beabstandelen Stellen frei von Klebstoff ist.

18. Endoskop nach Anspruch 17, **dadurch gekennzeichnet, daß** der Klebstoff in Form von gleichmäßig um den Umfang der Stablinse (12) verteilten Klebepunkten (36, 38; 54, 56) je Klebestelle aufgetragen ist.

19. Endoskop nach Anspruch 17 oder 18, **dadurch gekennzeichnet, daß** an der Außenseite der Stablinse (12) elastische Zentrierelemente (66) angebracht sind, die mit der Innenseite des Rohres (24) verklebt sind.

20. Endoskop nach Anspruch 17 oder 18, **dadurch gekennzeichnet, daß** eine als Knochenlinse (70) ausgebildete Stablinse mit radialen Verdickungen (72) vorgesehen ist, und daß die elastischen Klebestellen (74, 76) benachbart zu den Verdickungen (72) angebracht sind.

## Claims

1. Method for mounting at least one rod lens (12, 70) in the interior of a tube (24) belonging to an endoscope (10) by immobilizing the rod lens (12, 70) in the interior of the tube (24) at at least two points spaced axially apart from one another, having the following steps:
- selecting the outside diameter (A) of the rod lens (12, 70) such that after introduction of the rod lens (12, 70) into the tube (24), an air gap (34) is created circumferentially between the outer side (30) of the rod lens (12, 70) and the inner side (32) of the tube (24); and
- positioning the outer side (30) of the rod lens (12, 70) at a defined radial spacing from the inner side (32) of the tube (24) by way of at least two centering aids that engage at at least two points spaced axially apart from one another on the outer side (30) of the rod lens (12, 70), each centering aid having centering elements (66) that are distributed uniformly around the circumference of the rod lens (12, 70),
**characterized by**
providing direct elastic adhesive joins between the outer side (30) of the rod lens (12, 70) and the inner side (32) of the tube (24) at said axially spaced points.

2. Method of Claim 1, **characterized in that** the radial spacing between the outer side of the rod lens (12, 70) and the inner side (32) of the tube (24) is selected so that it lies in the range from about 0.05 to 0.3 mm, preferably in the area of about 0.1 mm.

3. Method of Claims 1 or 2, **characterized in that** positioning is accomplished with at least three centering elements, arranged with a circumferential offset of approximately 120°, for each centering aid.

4. Method of anyone of Claims 1 through 3, **characterized in that** the centering elements (66) are left in the assemblage of tube (24) and rod lens (12) after the adhesive join has been effected.

5. Method of anyone of Claims 1 through 3, **characterized in that** elongated, in particular cylindrical, bodies (52) are selected for positioning, and are set in place extending axially between the inner side (32) of the tube (24) and the outer side (30) of the rod lens (12); and they are removed again after the adhesive join is effected or they are left in the assemblage.

6. Method of anyone of Claims 1 through 3, **characterized in that** radially extending centering pins (40; 46), which pass through openings (42) in the wall of the tube (24) and are removed after the adhesive join is effected, are used for positioning.

7. Method of anyone of Claims 1 through 6, **characterized in that** at least one opening (44) in the wall of the tube (24), through which the adhesive for effecting the adhesive join is introduced between the tube (24) and the rod lens (12), is provided at each adhesive point.

8. Method of Claim 7, **characterized in that** three openings (44) distributed uniformly around the circumference of the tube (24), through which the adhesive for effecting the adhesive join between the tube (24) and the rod lens (12) is introduced in the form of adhesive dots (36, 38), are provided.

9. Method of Claim 7, **characterized in that** the adhesive for effecting the adhesive join between the tube (24) and the rod lens (12) is applied through the opening (44) in the form of an adhesive ring that encloses the circumference of the rod lens (12) in the region of the adhesive point.

10. Method of anyone of Claims 7 through 9, **characterized in that** the centering pins (46) pass through those openings (44) through which the adhesive is also applied; the outer side of the centering pins is made of a material that repels the adhesive or is coated with such a material; and they can be removed again from the assemblage after the adhesive join has been effected.

11. Method of Claim 10, **characterized in that** centering pins (46) that have conical tips (48) are used.

12. Method of anyone of Claims 1 through 3, **characterized in that** adhesive dots (54, 56) that expand or deform by activation are applied as centering elements onto the outer side (30) of the rod lens (12) or onto the inner side (32) of the tube (24).

13. Method of Claim 12, **characterized in that** the activation of the adhesive is accomplished by a heat treatment.

14. Method of Claims 12 or 13, **characterized in that** the adhesive dots (54, 56) made of adhesive are surrounded by a material or a surface that serves as a barrier to expansion of the adhesive.

15. Method of anyone of Claims 1 through 4, **characterized in that** elastic centering elements (66) that are adhesively bonded to the inner side (32) of the tube (24) are applied on the outer side of the rod lens (12).

16. Method of anyone of Claims 1 through 15, **characterized in that** in the case of a rod lens configured as a dog-bone lens (70) having radial enlargements (72), the adhesive join (74, 76) is applied adjacent to the enlargement (72).

17. Endoscope (10) having a tube (24) in the interior of which at least one rod lens (12) is immobilized at at least two points spaced axially apart from one another, the rod lens (12) having an outside diameter (A) dimensioned such that after introduction of the rod lens (12) into the tube (24), an air gap (34) is present between the outer side (30) of the rod lens (12) and the inner side (32) of the tube (24), and the outer side (30) of the rod lens (12) being arranged at a defined radial spacing from the inner side (32) of the tube (24), **characterized in that** the outer side (30) of the rod lens (12) is immobilized at said axially spaced points on the inner side (32) of the tube (24) via direct elastic adhesive points using adhesive, a space between said axially spaced points is free from adhesive.

18. Endoscope of Claim 17, **characterized in that** the adhesive is applied in the form of adhesive dots (36, 38; 54, 56), distributed uniformly around the circumference of the rod lens (12), for each adhesive point.

19. Endoscope of Claims 17 or 18, **characterized in that** elastic centering elements (66) that are adhesively bonded to the inner side of the tube (24) are applied on the outer side of the rod lens (12).

20. Endoscope of Claims 17 or 18, **characterized in that** a rod lens configured as a dog-bone lens (70) is equipped with radial enlargements (72); and the elastic adhesive points (74, 76) are applied adjacent to the enlargements (72).

## Revendications

1. Procédé pour le montage d'au moins une lentille cylindrique (12, 70) à l'intérieur d'un tube (24) faisant partie d'un endoscope (10) par fixation de la lentille cylindrique (12, 70) à l'intérieur du tube (24) en au moins deux endroits distants l'un de l'autre dans le sens axial, comprenant les étapes suivantes :
- choix du diamètre extérieur (A) de la lentille cylindrique (12, 70) de façon que, après la pose de la lentille cylindrique (12, 70) dans le tube (24), un interstice d'air (34) soit formé à la périphérie entre la face extérieure (30) de la lentille cylindrique (12, 70) et la face intérieure (32) du tube (24), et
- positionnement de la face extérieure (30) de la lentille cylindrique (12, 70) à une distance radiale définie par rapport à la face intérieure (32) du tube (24) par au moins deux dispositifs auxiliaires de centrage qui s'appliquent en au moins deux endroits, distants l'un de l'autre dans le sens axial, de la face extérieure (30) de la lentille cylindrique (12, 70), chaque dispositif auxiliaire de centrage présentant des éléments de centrage (66) qui sont répartis régulièrement autour de la périphérie de la lentille cylindrique (12, 70),
**caractérisé par** la réalisation d'assemblages collés élastiques directs entre la face extérieure (30) de la lentille cylindrique (12, 70) et la face intérieure (32) du tube (24) aux endroits distants l'un de l'autre dans le sens axial.

2. Procédé selon la revendication 1, **caractérisé en ce que** l'écart radial entre la face extérieure de la lentille cylindrique (12, 70) et la face intérieure (32) du tube (24) est choisi de façon qu'il se situe dans la plage d'environ 0,05 à 0,3 mm, de préférence dans la plage d'environ 0,1 mm.

3. Procédé selon la revendication 1 ou 2, **caractérisé en ce que** le positionnement est effectué avec trois éléments de centrage par dispositif auxiliaire de centrage, qui sont disposés sur la périphérie de manière décalée d'environ 120°.

4. Procédé selon l'une des revendications 1 à 3, **caractérisé en ce qu'**on laisse en place les éléments de centrage (66) dans l'assemblage du tube (24) et de la lentille cylindrique (12) après la réalisation de l'assemblage collé.

5. Procédé selon l'une des revendications 1 à 3, **caractérisé en ce que** l'on choisit, pour le positionnement, des corps (52) allongés, en particulier cylindriques, qui sont posés en s'étendant entre la face intérieure (32) du tube (24) et la face extérieure (30) de la lentille cylindrique (12) et **en ce qu'**on enlève ceux-ci après la réalisation de l'assemblage collé ou on les laisse en place dans l'assemblage.

6. Procédé selon l'une des revendications 1 à 3, **caractérisé en ce qu'**on utilise, pour le positionnement, des goupilles de centrage (40 ; 46) s'étendant dans le sens radial, qui pénètrent par des ouvertures (42) dans la paroi du tube (24) et qui sont enlevées après la réalisation de l'assemblage collé.

7. Procédé selon l'une des revendications 1 à 6, **caractérisé en ce qu'**il est prévu, à chaque emplacement de collage, au moins une ouverture (44) dans la paroi du tube (24), au travers de laquelle la colle est appliquée pour réaliser l'assemblage collé entre le tube (24) et la lentille cylindrique (12).

8. Procédé selon la revendication 7, **caractérisé en ce qu'**il est prévu trois ouvertures (44) réparties régulièrement autour de la périphérie du tube (24), au travers desquelles la colle est appliquée sous forme de points de colle (36, 38) pour réaliser l'assemblage collé entre le tube (24) et la lentille cylindrique (12).

9. Procédé selon la revendication 7, **caractérisé en ce que** la colle destinée à réaliser l'assemblage collé entre le tube (24) et la lentille cylindrique (12) est appliquée au travers de l'ouverture (44) sous la forme d'une bague de collage qui entoure la périphérie de la lentille cylindrique (12) dans la zone de l'endroit de collage.

10. Procédé selon l'une des revendications 7 à 9, **caractérisé en ce que** les goupilles de centrage (46) pénètrent à travers les ouvertures (44) au travers desquelles la colle est aussi appliquée, **en ce que** la face extérieure des goupilles de centrage est réalisée dans une matière refusant la colle ou enduite d'une telle matière et **en ce que** les goupilles de centrage sont retirées de l'assemblage après la réalisation de l'assemblage collé.

11. Procédé selon la revendication 10, **caractérisé en ce qu'**on utilise des goupilles de centrage (46) qui présentent une pointe conique (48).

12. Procédé selon l'une des revendications 1 à 3, **caractérisé en ce que**, comme éléments de centrage, on applique sur la face extérieure (30) de la lentille cylindrique (12) ou sur la face intérieure (32) du tube (24), des points de colle (54, 56) qui s'étirent ou se déforment par activation de la colle.

13. Procédé selon la revendication 12, **caractérisé en ce que** l'activation de la colle a lieu par traitement thermique.

14. Procédé selon la revendication 12 ou 13, **caractérisé en ce que** les points de colle (54, 56) constitués de colle sont entourés d'une matière ou d'une surface qui sert de barrière à l'étirement de la colle.

15. Procédé selon l'une des revendications 1 à 4, **caractérisé en ce que** des éléments de centrage élastiques (66), qui sont collés à la face intérieure (32) du tube (24), sont posés sur la face extérieure de la lentille cylindrique (12).

16. Procédé selon l'une des revendications 1 à 15, **caractérisé en ce que**, dans le cas d'une lentille cylindrique en forme d'os (70) avec des épaississements radiaux (72), l'assemblage collé (74, 76) est disposé de manière adjacente auxdits épaississements (72).

17. Endoscope (10) avec un tube (24) à l'intérieur duquel au moins une lentille cylindrique (12) est fixée en au moins deux endroits distants l'un de l'autre dans le sens axial, la lentille cylindrique (12) présentant un diamètre extérieur (A) qui est dimensionné de façon qu'il existe un interstice d'air (34) entre la face extérieure (30) de la lentille cylindrique (12) et la face intérieure du tube (24) après la pose de la lentille cylindrique (12) dans le tube (24), et la face extérieure (30) de la lentille cylindrique (12) étant disposée à une distance radiale définie par rapport à la face intérieure (32) du tube (24), **caractérisé en ce que** la face extérieure (30) de la lentille cylindrique (12) est fixée par des points de collage élastiques directs au moyen de colle sur la face intérieure (32) du tube (24) en des endroits distants les uns des autres, l'espace entre les endroits distants les uns des autres dans le sens axial étant exempt de colle.

18. Endoscope selon la revendication 17, **caractérisé en ce que** la colle est appliquée, en chaque endroit de collage, sous la forme de points de colle répartis régulièrement sur la périphérie de la lentille cylindrique (12).

19. Endoscope selon la revendication 17 ou 18, **caractérisé en ce que** des éléments de centrage élastiques (66), qui sont collés à la face intérieure du tube (24), sont collés sur la face extérieure de la lentille (12).

20. Endoscope selon la revendication 17 ou 18, **caractérisé en ce qu'**une lentille cylindrique en forme d'os (70) est prévue avec des épaississements radiaux (72) et **en ce que** les endroits de collage élastiques (74, 76) sont appliqués de manière adjacente auxdits épaississements (72).
